# EUROPEAN PATENT APPLICATION

(11) **EP 1 018 326 A1**
(43) Date of publication of application: **12.07.2000**
(21) Application number: 99100118.1
(22) Date of filing: 04.01.1999
(51) Int. Cl.: A61F 5/451

(54) **Container for the collection of bodily waste provided with an elastic flange insert**

(71) Applicant: THE PROCTER & GAMBLE COMPANY, Cincinnati, Ohio 45202 (US)
(72) Inventor: d'Acchioli, Vincenzo, 65779 Kelkheim/Ts (DE); Allen, Patrick Jay, Cincinnati, Ohio 45249 (US); Evangelista, Olindo, 66023 Francavilla al Mare, Chieti (IT); Palumbo, Gianfranco, 61348 Bad Homburg (DE)
(74) Representative: Kohol, Sonia

(57) **Abstract**

The present invention relates to human waste management devices, which comprise a storage means, and to faecal management devices, all for babies, children or adults. The invention resides principally in providing the device with an elastic insert for the flange (12).

## Description

### Field of the invention

The present invention relates to human waste management devices for babies, children or adults. Said devices are provided with an elastic insert for the flange.

### Background of the invention

Human waste management devices are known articles of manufacture that are designed to be worn principally by incontinence sufferers and in particular by bedridden patients. Such human waste management devices are attached to the perianal or uro-genital region of the wearer and are intended to entrap and immediately contain faecal material, urine and other bodily discharges. Such devices, as they are mostly known today are constituted of a bag at one extremity of which is positioned the aperture and the attachment device, which typically is adhesive.

For example faecal management devices are disclosed in e. g. the following documents: US 3,577,989, which details a disposable plastic bag for incontinence sufferers. US 4,784,656 describes a receptacle for collecting faecal matter The receptacle is formed from two sheets of thermoplastic film that are heat sealed along their side edges. GB 2 152 387, which teaches a faecal collector for incontinence sufferers comprising a collection bag consisting of a pair of panels of thermoplastic sheet material joined at their margins.

EP 245 064 discloses bags substantially consisting of a front and a rear wall, which are made of a synthetic plastic material, such as PVC. GB 2 215 605 discloses an ostonomy bag comprising panels of synthetic plastic material, the rear bag wall further comprising a needled film.

Urine management devices are, for instance, disclosed in the following documents: GB 1,092,274 discloses a pediatric urine collector for female use comprising a collector bag of plastic material opening. The base of the opening is provided with a wedge like projection adapted to engage the lower perineal area of the infant. The collector is secured to the body of the wear by adhesive material. GB 2,268,882 discloses a urostomy pouch/bag of plastic material provided with a circular stomal orifice which is surrounded by a first coupling member, by which the pouch can be affixed to a counterpart coupling member, which can be attached to a wearer. The pouch may also be provided for use as a kit further comprising an applicator of super absorbent material which can be injected into the pouch by the use of a plunger. US 4,804,377 discloses a collector for urine specimens from children. The collector comprises a rectangular flange for adhesive attachment, which comprises a round, slightly oval aperture. EP 140 478 discloses a disposable diaper having a water proof barrier preferably polypropylene or polyethylene formed as a flattened bag having a single opening. US 1,092,274 discloses a urine collector for female infants comprising an aperture which is smaller at the bottom end than at the top end and could be described as droplet shaped. US 3,292,626 also discloses a urine collector for female infants comprising a circular or droplet shaped aperture. Chinese patent application CN 1079381 discloses urine bags for infants with circular and elliptical apertures.

Many wearers, who make use of human waste management devices have sensitive skin due to their age, whether very old or very young, and furthermore sometimes also suffer from skin irritations. Thus, it is important to minimise skin contact with faecal matter or urine. To this end the aperture should not be chosen to large, since the area of the aperture limits the skin area which potentially comes in contact with faecal matter or urine.

Another problem associated with human waste management devices is their behaviour after detachment, sometimes even unintentional detachment, and their handling after detachment. Since they regularly are a source of malodour and possibly of leakage, the area of the aperture should not be chosen larger than necessary for good performance.

Thus, considering skin protection and the handling after use, a small aperture is desirable. On the other hand, with regard to the importance of easy and proper placement of the device a large aperture is desirable.

Proper placement of the device is a key issue in the field of human waste management devices. Total or substantial misplacement of the device will lead to a severe misfunctioning, in particular incomplete collection of urine or faecal matter and leaking. Placement is also important with regard to reliable adhesion. If the aperture of the human waste management device is not sufficiently in registry with the urinary duct or anal opening of a wearer, the adhesive on the flange may come into contact with urine or faecal matter to an undesirable extent. With some adhesives this can lead to the detachment of the adhesively secured device, obviously entailing the most unwanted consequences. Detachment may also be caused by pressure building up, namely in the defaecation process, when the device is not sufficiently in registry with the anal opening. Unintentional detachment is known in the art, as mentioned in GB 2 116 849.

If the misplacement of the device is recognised before use, the placement of the device is normally corrected. The necessary detachment and reattachment of the device means an additional stress on the skin of the wearer. Many wearers, who make use of urine management devices have a sensitive skin due to their age, whether very old or very young, and furthermore sometimes also suffer from skin irritations. Proper placement of the device in the first place is therefore highly desirable.

Hence, there still exists a need for a human waste management devices and in particular for a faecal management devices which can be easily and correctly positioned onto the desired area of the wearer by the caretaker or wearer themselves without causing discomfort to the wearer. There also is a need for a device which optimal anatomical fit and reliable adhesive attachment. Furthermore, there is a need for a human waste management device which limits the risk of skin irritation.

In attempting to overcome all the aforementioned problems relating to the prior art, it has now been found that adhesive human waste management devices can be designed which have excellent ease of placement and highly improved skin protection properties, through the use of a simple, but efficient device. The same design does not only greatly help in placing the device but also assists in handling after detachment.

### Summary of the invention

The present invention relates to human waste management devices for babies, children or adults. The invention resides principally in providing such devices with an elastic insert for the flange.

### Brief description of the drawings

It is believed that the invention will be better understood from the foregoing description in conjunction with the accompanying drawings in which:
Figure 1 is a perspective view of a preferred embodiment of a faecal management device (10) according to the present invention.
Figure 2 is a side view of another preferred embodiment of a flange (12) for a faecal management device (10).
Figure 3 is a top plan view of the preferred embodiment of a flange (12) for a faecal management device (10) as shown in Figure 2.

### Detailed description of the invention

The invention relates to human waste management devices. Such a human waste management device may be a faecal management device (10), which is designed for attachment to the anal area and mainly used for collecting faeces, or it may be a urine management device, which is attached to the urinary duct and mainly used for collecting urine. All references to urine management devices as made herein refer to devices which comprise a storage means such as a bag and not to devices which do not have any storage capacity and typically serve to close the urethra. A human waste management device may also be a device to collect both urine and faeces and thus be attached to both of the above areas. All of the above human waste management devices are preferably designed for single use and disposal thereafter.

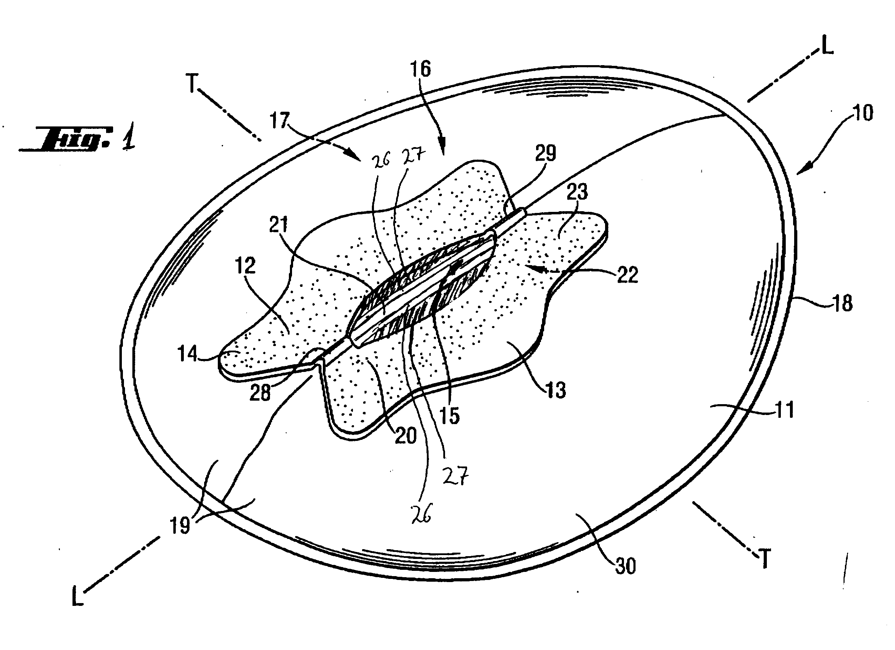

A faecal management device (10) is shown in Figure 1.

### Description of the human waste management device as a whole

Typically human waste management devices comprise a bag (11) having an aperture (21) and a flange (12) surrounding the aperture for preferably adhesive attachment to the perianal area of a wearer as visible from Figure 1. Any human waste management device known in the art can be provided according to the present invention.

The bag (11) as used herein is a flexible receptacle for the containment of excreted faecal matter or urine. The bag (11) is designed to safely contain any entrapped material, typically it will be liquid impermeable, yet it may be breathable. The bag (11) is designed of sufficient strength to withstand rupture in use, also when pressure on the bag (11) is exerted in typical wearing conditions, such as sitting.

According to the present invention the bag material can comprise one or multiple layers, preferably two or three layers. The layer on the inside of the bag (11), which will typically at least partially come in contact with faecal material or urine is called the inner layer. The outermost layer of the bag, which will typically at least partially come in contact with the skin to the wearer and the garments of the wearer, is called the outer layer.

The layers of the bag material may comprise any material, preferably so that the bag is liquid impervious. The preferred optical properties of any such material will be described below in more detail. The layers may in particular comprise any material such as non-wovens or films. In a preferred embodiment of the present invention a laminate may be formed from a non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag material may comprise a non-woven layer. Such material layers present an uneven surface to the skin of the wearer and thus reduce significantly the problem of occlusion and greatly improve skin healthiness.

In one preferred embodiment of the present invention the bag material comprises two layers. Preferably the outer layer comprises a non-woven layer and the inner layer comprises a film.

In yet another preferred embodiment of the present invention, the bag material comprises three layers, preferably one film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a film and the other two layers comprise non-wovens.

The non-woven layer or the non-woven layers comprised by the bag material may be hydrophobic or hydrophilic. If the bag material does not comprise a film layer, preferably at least one non-woven layer is hydrophobic. As a consequence, fluid penetration is resisted through the wearer facing portion (16) and the garment facing portion (17) of the human waste management device (10). If the bag material comprises a film or a hydrophobic non-woven layer, further non-woven layers may be hydrophilic.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, non-anionic, cationic and non-cationic surfactants, may be added to further enhance softness and surface smoothness.

Furthermore, the non-woven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

According to the present invention, depending on the shape of the bag (11) required, the bag (11) may be provided from a unitary piece of material or a number of separate pieces of material, which may be identical or different and which are sealed at their respective peripheries. The preferred shape of the bag depends in particular on the intended use thereof, i.e. whether the device is intended for bedridden patients or active patients suffering from incontinence or requiring an artificial bowel or for infants.

The bags described herein preferably have a wearer facing portion (16) and a garment facing portion (17), which both comprise separate pieces of material. The wearer facing portion (16) and the garment facing portion (17) are sealed at the periphery of the bag (11), thus creating a bag peripheral rim (18). The wearer facing portion (16) and the garment facing portion (17) may each independently comprise more than one section of material. Preferably the garment facing portion (17) comprises only one section of material; most preferably also the wearer facing portion (16) comprises only one section of material.

The wearer facing portion (16), the garment facing portion (17) and the pieces of material comprised by either of these portions are secured to each other by means known to the man skilled in the art, such as adhesive, thermobonding or pressure bonding in order to provide the desired bag configuration. The rim (18), at which the wearer facing portion (16) and the garment facing portion (17) are sealed together, may be provided inside the bag (11) rather than outside the bag (11), thus being coextensive with the inner surface (15) of the bag (11) rather than with the outer surface (30) of the bag (11).

Hence a variety of shapes of the bag is within the scope of the present invention. Particularly, preferred shapes are flat circular type bags, cone shaped bags, truncated cone shaped bags and pyramidal or truncated pyramidal shaped bags and flat T shaped bags.

In one embodiment of the present invention the bag (11) may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag or may be secured to the inner surface (15) of the bag (11). Any known techniques for securing absorbent material to nonwoven and film substrates may be used to secure the absorbent material to the inner surface (15) of the bag. The absorbent material may also be arranged to have any desired shape or configuration (e.g., rectangular, oval, circular, etc.).

The bag (11) is provided with an aperture (21) whereby faecal matter or urine is received from the body prior to storage within the bag cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction or in both directions, e.g. the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange is attached to the bag by adhesive. Typically, the bag will be attached to the flange, towards the outer periphery of flange so as not to cause any obstruction for the entering faecal matter or urine.

The flange may be provided in any size depending on the wearer group for which the device is intended. Similarly the flange may be provided in any shape and preferably has a symmetrical shape preferably comprising a plurality of lobes (13).

The flange comprises a garment facing portion (22) and a wearer facing portion (23). In an preferred embodiment these are two large, substantially flat surfaces, however, the flange (12) may also comprise projections, a front projection (28) and/or a rear projection (29), in case of a faecal management device (10) designed to fit the perineal and/or coccygeal area of the wearer and in case of a urine management device (10) designed to fit the genital and/or perineal area.

The flange (12) should be made of soft, flexible and malleable material to allow easy placement of the flange (12) to the perianal or uro-genital area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/m², more preferably 50 g/m². Other thermoplastic foam materials, or other suitable plastics sheet materials having the described properties of such foams (i.e., softness, pliability, stretchability, and contractability) might also be used.

According to the present invention the human waste management device (10) further comprises an attachment means to secure the device to the wearer. Such means include straps and more preferably comprises a body-compatible pressure sensitive adhesive (20) applied to the wearer facing portion (23) of the flange (12).

The adhesive (20) is preferably covered with a release means (not shown) in order to protect the adhesive (20), such as siliconized paper. The adhesive (20) can cover the entire wearer facing portion (23) of the flange (12), more preferably the flange (12) has at least one, preferably two to six non-adhesive portions. These portions may be adhesive free or may contain inactivated or covered adhesives. As is evident from Figure 1, the adhesive is in one preferred embodiment not applied to the entire wearer facing portion (23) of the flange (12), so as to provide lobes (13) on either side of the flange (12) which are non-adhesive and can thereby serve to facilitate placement and removal of the device whilst avoiding contact with the adhesive. These lobes (13) are however preferably also covered by the release means. Before application of the human waste management device (10) to the skin of the wearer, the release means if present is removed.

According to the present invention any medically approved water resistant pressure sensitive adhesive may be used to attach the device to the perianal or uro-genital area of the wearer, such as hydrocolloid adhesives and hydrogel adhesives. Particularly effective adhesives in providing the desired adhesive properties to secure the flange to the skin of the wearer at the sensitive perianal area, whilst allowing for relatively painless application and removal, are formed from crosslinking polymers with a plastisicer to form a 3-dimensional matrix.

The adhesive (20) can be applied to the wearer facing portion (23) of the flange (12) by any means known in the art such as slot coating, spiral, or bead application or printing. Typically the adhesive (20) is applied at a basis weight of from 20g/m² to 2500g/m², more preferably from 500g/m² to 2000g/m² most preferably from 700g/m² to 1500g/m² depending on the end use envisioned. For example, for human waste management devices (10) to be used for babies the amount of adhesive (20) may be less than for human waste management devices (10) designed for active adult incontinence sufferers.

The human waste management device (10) of the present invention has been found to be particularly useful and beneficial when used in conjunction with a garment, or diaper (50), preferably a disposable diaper.

According to the present invention the human waste management device is provided with an elastic insert (26) for the flange (12). Preferably the flange (12) is additionally provided with a skirt (27), for example a non-woven material, which extends into and closes parts of the aperture (21) defined by the inner periphery (25) of the flange (12) when the human waste management device does not receive faecal matter or urine to thereby provide a slit.

The elastic inserts (26) can be provided from any material suitable for contact with faecal matter and the human skin and providing an elastic modulus as described below. A preferred elastic material for the elastic inserts (26) is natural rubber. In one preferred embodiment each elastic insert (26) comprises four threads of natural rubber which are substantially circular and preferably have a diameter from 0.05 mm to 1 mm, preferably 0.1 mm to 0.25 mm.

The flange (12) may comprise any number of elastic inserts (26). Preferably the flange (12) comprises one to four elastic inserts (26). Preferably the elastic inserts (26) are provided between two points on the inner periphery of the flange (12) so that they span across the aperture (21). More preferably the elastic inserts (26) are oriented in the longitudinal direction.

The elastic inserts (26) may be attached to the flange (12) by any means known in the art, such as thermobonding, ultrasonic bonding, stitching and adhesive. The elastic inserts (26) may also be attached to skirt (27) - either in addition to attachment to the flange (12) or without further attachment to the flange (12).

The skirt can be made of any soft, pliable material, which allows for opening and closing of the slit. Preferably the skirt (27) is provided from a non-woven material. In contrast to the flange (12) the skirt (27) is preferably not provided with an adhesive. In one preferred embodiment the garment facing portion (22) of the flange (12) is provided with a non-woven layer which extends into the aperture (21) so as to provide a skirt (27) between the two elastic inserts (26) and the aperture (21).

The skirt (27) may cover any portion of the aperture (21). Preferably the skirt (27) is attached to the flange (12). More preferably the skirt (27) is in addition attached to the elastic inserts (26). Most preferably the skirt (27) extends from the flange (12) to the elastic inserts (26) so that only one area of the aperture (21) is left open when no faecal matter is received. Most preferably this open area has the form of a slit.

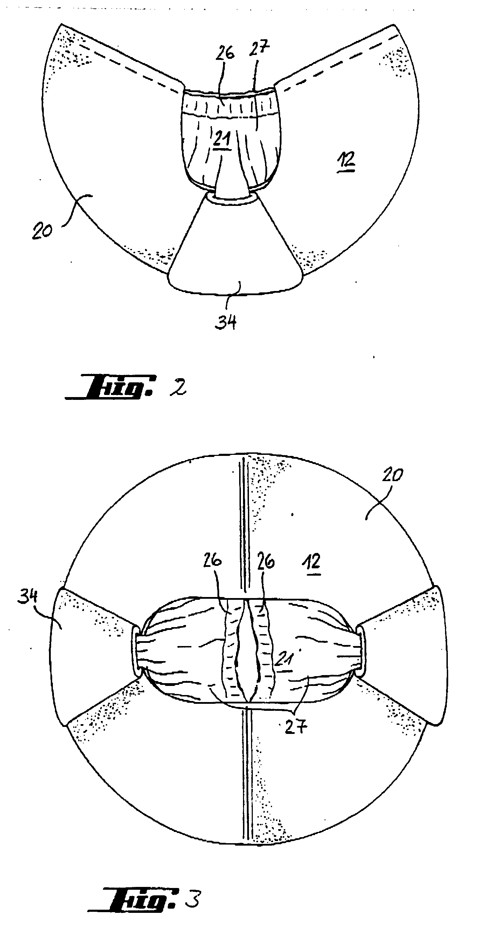

Figures 1 shows a preferred embodiment of the present invention for a faecal management device (10). Figures 2 and 3 show another preferred embodiment of the present invention for a faecal management device (10).The elastic insert (26) is provided by two parallel elastic threads in the longitudinal direction. The device (10) further comprises a skirt (27), which extends from the inner periphery of the flange (12) to the elastic inserts (26), such that the only portion of the aperture (21) which is not covered by the skirt (27) has the form of a slit oriented in the longitudinal direction.

When the faecal management device (10) is worn in the intended wearing position said slit is in registry with the anal opening, essentially oriented along the anal groove, while the aperture (21) of the faecal management device (10) surrounds the anal opening and has a larger distance to the anal opening than the elastic inserts (26). In the defaecation process pressure builds up on the elastic inserts (26), while no pressure builds up affecting the aperture (21), which could contribute to an unintentional detachment of the device (10).

Due to their elastic properties the elastic inserts (26) will expand when they are subject to pressure in the defaecation process. Thereby the slit will open so as to receive faecal matter which is then transmitted into the bag (11). Upon completion of the defaecation process, the elastic inserts (26) will relax and thus go back to their initial position forming a slit. After the reclosure of the slit faecal matter, which is then entrapped in the bag (11), is prevented from coming into skin contact in areas other than the slit, since other portions of the aperture (21) are provided with the skirt (27). This largely helps to reduce skin irritations.

The performance of the device inter alia depends of the width of the slit when the elastic inserts (26) are not expanded and also on the width of the opening when the elastic inserts (26) are expanded. The slit, when not expanded, should not be too wide in order to achieve good skin protection. On the other hand the slit should not be too small as not to hinder the entrapping of faecal material, which ideally is received in a central position between the two elastic inserts (26). It has been found that the slit, when not expanded, preferably has a transversal diameter from 20 mm to 0.1 mm, more preferably from 10 mm to 1 mm, yet more preferably from 4 mm to 2 mm. When the elastic inserts (26) are fully expanded, without being overexpanded, i.e. without damage being caused to the elastic inserts (26), the transversal diameter of the opening should be 60% to 100%, more preferably 80% to 90% of the transversal diameter of the aperture (21). For faecal management devices (10) for babies the transversal diameter of the opening when the elastic inserts (26) are expanded should be between 20 mm and 50 mm, more preferably between 30 mm and 40 mm.

The elastic modulus of the elastic inserts (26) depends on the intended use of the human waste management device, for example whether it is a faecal management device or a urine management device and whether it is intended for use for adults or infants. Suitable elastic materials have an elastic modulus at 200% of elongation between 0.1 N/mm² and 100 N/mm², more preferably between 1 N/mm² and 10 N/mm².

As can be seen from Figure 2 the flange (12) in its relaxed configuration has a three dimensional configuration which is curved so as to follow the curved line of the human anatomy which extends from the coccyx via the anal opening to the perineum, i.e. the wearer facing portion (23) of the flange (12) comprises a concave curve as seen from the wearer facing side. This curved configuration has been found to provide an improved anatomical fit of the device (10). In particular when a soft, pliable material is used for the flange (12) the elastic inserts (26) have been found to be useful in supporting the longitudinal curved configuration of the flange (12). Elastic inserts (26) oriented in the longitudinal direction are most beneficial for this. Without the use of elastic inserts (26) the intended shape of the flange (12), namely during application of the device (10), would only be supported by the rigidness of the flange material. A rigid flange material, however, may not be ideal for good wearing comfort.

## Claims

1. A human waste management device comprising a bag (11), said bag (11) having an aperture (21) and a flange (12) surrounding said aperture (21), said flange (12) comprising at least one elastic insert (26).

2. Human waste management device according to Claim 1, characterised in that said flange (12) further comprises a skirt (27).

3. Human waste management device according to any one of the preceding claims, characterised in that said flange (12) comprises two elastic inserts (26) which are parallel.

4. Human waste management device according to Claim 3, characterised in that said flange (12) further comprises a skirt (27) which forms a slit within said aperture (21).

5. Human waste management device according to Claim 4, characterised in that said slit is oriented in the longitudinal direction.

6. Human waste management device according to any one of the preceding claims, characterised in that said flange (12) comprises at least one elastic insert (26).

7. Human waste management device according to any one of the preceding claims, characterised in that said flange (12) has a relaxed configuration, characterised in that said relaxed configuration is three dimensional.

8. Human waste management device according to Claim 7, said flange (12) having a wearer facing portion (23) and a longitudinal direction and a transversal direction, characterised in that said wearer facing portion (23) of said flange (12) comprises a concave curve in said longitudinal direction.
